# FASCICULE DE BREVET EUROPEEN

(11) **EP 2 130 040 B1**
(45) Date de publication et mention de la délivrance du brevet: **13.02.2013**
(21) Numéro de dépôt: 08787791.6
(22) Date de dépôt: 14.03.2008
(51) Int. Cl.: G01N 33/10, G01N 3/10, G01N 11/00

(54) **DISPOSITIF D'ANALYSE PAR SOUFFLAGE DES CARACTERISTIQUES D'UN ECHANTILLON DE PATE ET EXTENSIMETRE POUR SA MISE EN OEUVRE**
VORRICHTUNG ZUR BLASUNGSANALYSE DER EIGENSCHAFTEN EINER PASTÖSEN PROBE UND EXTENSIOMETER ZU IHRER ANWENDUNG
DEVICE FOR THE BLOW ANALYSIS OF THE CHARACTERISTICS OF A PASTE SAMPLE, AND EXTENSIOMETER FOR IMPLEMENTING SAME

(30) Priorité: 16.03.2007 FR 0701911
(43) Date de publication de la demande: 09.12.2009
(73) Titulaire: Chopin Technologies, 92396 Villeneuve La Garenne Cedex (FR)
(72) Inventeur: DUBAT, Arnaud, F-78112 Fourqueux (FR); LECOMTE, Emmanuel, F-78400 Chatou (FR)
(74) Mandataire: Derambure, Christian
(86) Numéro de dépôt international: PCT/FR2008/000334
(87) Numéro de publication internationale: WO 2008/132344

(56) Documents cités:
- EP-A- 1 443 317
- FR-A- 525 686
- JP-A- 8 313 422
- US-A- 4 838 081

## Description

L'invention concerne un procédé d'analyse par soufflage, des caractéristiques d'un échantillon de pâte ainsi qu'un extensimètre pour la mise en oeuvre d'un tel procédé.

Il est connu d'analyser les caractéristiques d'un échantillon de pâte au moyen d'un extensimètre également connu sous la dénomination commerciale d' « alvéographe » inventé par Monsieur CHOPIN et décrit notamment dans le document FR-525 686 A.

Dans un tel dispositif, un disque de pâte faite à partir d'une farine est pincé sur son pourtour, puis soumis à une pression de gaz sur sa face inférieure. Le disque se déforme en une bulle vers le haut dont le volume croît tandis que la paroi s'amincit jusqu'à éclatement.

Un manomètre enregistre la pression en fonction du temps, c'est-à-dire la déformation.

On obtient ainsi une courbe ou diagramme à partir duquel on peut déterminer diverses valeurs indicatives telles que la ténacité et l'extensibilité de la farine, le travail de déformation et la configuration du diagramme permettant en outre d'évaluer l'aptitude de la farine à une utilisation donnée.

Ce dispositif connu est assez satisfaisant quant aux résultats obtenus mais il présente divers inconvénients quant à sa robotisation, son nettoyage après éclatement de l'échantillon et le bouchage inévitable de l'orifice de soufflage par l'échantillon lui-même après son éclatement, etc.

Par ailleurs, la bulle ainsi soufflée ne présente pas une sphéricité parfaite ce qui peut nuire aux résultats.

Même si les moyens ont un peu évolué depuis son invention par Monsieur CHOPIN, on a toujours procédé jusqu'à présent par soufflage ou surpression de la face inférieure du disque de pâte afin de former une bulle vers le haut.

Dans le document EP-1 443 317 A par exemple, de plus de quatre-vingts ans postérieur aux dispositif et procédé CHOPIN précités, on décrit aussi un dispositif et un procédé qui consistent à réaliser une bulle en établissant une surpression relative sur la face inférieure de l'échantillon de pâte. On retrouve donc ici au moins certains des mêmes inconvénients précités et en particulier, une sphéricité imparfaite de la bulle ainsi obtenue.

Il est vrai que dans ce document EP-1 443 317 A, on décrit aussi selon une variante, un procédé particulier selon lequel on fait osciller ou vibrer l'échantillon de pâte en effectuant de manière répétitive des pressions et dépressions relatives alternativement d'un côté à l'autre de l'échantillon de pâte.

Dans ce mode de réalisation, il n'est cependant pas question de faire une bulle et donc encore moins de gonfler celle-ci jusqu'à son éclatement.

Les inventeurs ont mis au point un procédé et un appareil qui permettent de pallier les inconvénients précités.

Le procédé d'analyse par soufflage selon l'invention part classiquement, comme décrit dans le document FR-525 686 A, d'un échantillon de pâte sous la forme d'un disque qui est positionné sensiblement à l'horizontal, qui est pincé sur son pourtour et qui est soumis à une pression de gaz pour former une bulle qui est gonflée jusqu'à éclatement pendant que l'on enregistre la pression exercée, mais ledit procédé est notamment remarquable en ce qu'il consiste à soumettre le disque de pâte à une pression de gaz sur sa face supérieure de telle sorte que la bulle se forme par extension, vers le bas.

Les inventeurs ont en effet découvert que la bulle ainsi soufflée vers le bas présente une meilleure sphéricité et ils ont ainsi vaincu un préjugé en inversant la manière classique de procéder depuis l'invention d'origine.

En outre, un tel procédé permet une robotisation facilitée comme il sera expliqué ci-après, un nettoyage aisé et évite tout encrassement de l'orifice de soufflage dont il est question également ci-après.

En effet, selon l'invention le disque de pâte est préalablement écrasé entre deux plateaux, respectivement inférieur et supérieur. L'invention préconise également que l'on effectue le soufflage par un orifice du plateau supérieur et que l'on amène le disque de pâte sous ledit plateau supérieur au moyen d'une platine support mobile que l'on positionne au centre d'une couronne complémentaire de ladite platine pour former avec celle-ci le plateau inférieur.

Dans ce cas par exemple, la platine support est mue selon deux mouvements combinés de rotation et de translation en vue de permettre son positionnement et son mouvement d'écrasement du disque de pâte, ainsi que son dégagement pour libérer l'espace sous une partie du disque de pâte afin de permettre son gonflage.

Outre le procédé mentionné ci-avant, l'invention propose un extensimètre muni classiquement de moyens pour pincer le disque de pâte sur son pourtour, d'un moyen de mise sous pression d'un gaz pour former par soufflage une bulle et de moyens d'enregistrement de la pression exercée, mais qui est notamment remarquable en ce qu'il comporte un plateau supérieur fixe muni d'un orifice de soufflage obturable et une couronne qui détermine un ajour central et qui est disposée sous le plateau supérieur, ladite couronne étant montée de manière translative par rapport audit plateau supérieur afin de pouvoir s'en rapprocher, être maintenue dans cette position, et respectivement s'en écarter, tandis qu'une platine support mobile est prévue pour transporter le disque de pâte sous ladite couronne, ladite platine support mobile étant dimensionnée pour venir dans l'ajour central de la couronne et compléter celle-ci afin de former avec elle un plateau inférieur destiné à permettre l'écrasement du disque de pâte en se rapprochant du plateau supérieur.

Avantageusement dans ce cas, la platine support est munie d'un bras actionné selon des mouvements de rotation et de translation de manière à permettre successivement, le positionnement de ladite platine sous la couronne et ensuite dans l'ajour central de celle-ci, puis sa translation avec ladite couronne vers le plateau supérieur pour assurer l'écrasement du disque de pâte, au moins un moyen étant prévu pour maintenir dans cette position la couronne, puis le dégagement de la platine support de ladite couronne afin de libérer ledit ajour central et l'espace situé sous une partie du disque de pâte qui peut ainsi rester pincé sur son pourtour entre le plateau supérieur et la couronne et subir un soufflage sur sa face supérieure par l'orifice dudit plateau supérieur.

Par exemple, le bras est entraîné en translation et en rotation par un actionneur à came et le moyen qui permet d'assurer le maintien en position rapprochée de la couronne par rapport au plateau supérieur, est prévu pour permettre aussi d'assurer l'éloignement de ladite couronne dudit plateau supérieur.

L'invention sera bien comprise et d'autres particularités apparaîtront à la lecture de la description qui va suivre et qui se réfère aux dessins annexés dans lesquels :
- la figure 1 montre en perspective un appareil selon l'invention, en position initiale,
- la figure 2 est une vue en élévation de l'appareil de la figure 1,
- la figure 3 est une coupe selon III-III de la figure 2 à plus grande échelle,
- les figures 4 à 13 sont des coupes semblables à la coupe de la figure 3 selon diverses phases successives de fonctionnement de l'appareil, correspondant aux diverses phases du procédé d'analyse selon l'invention.

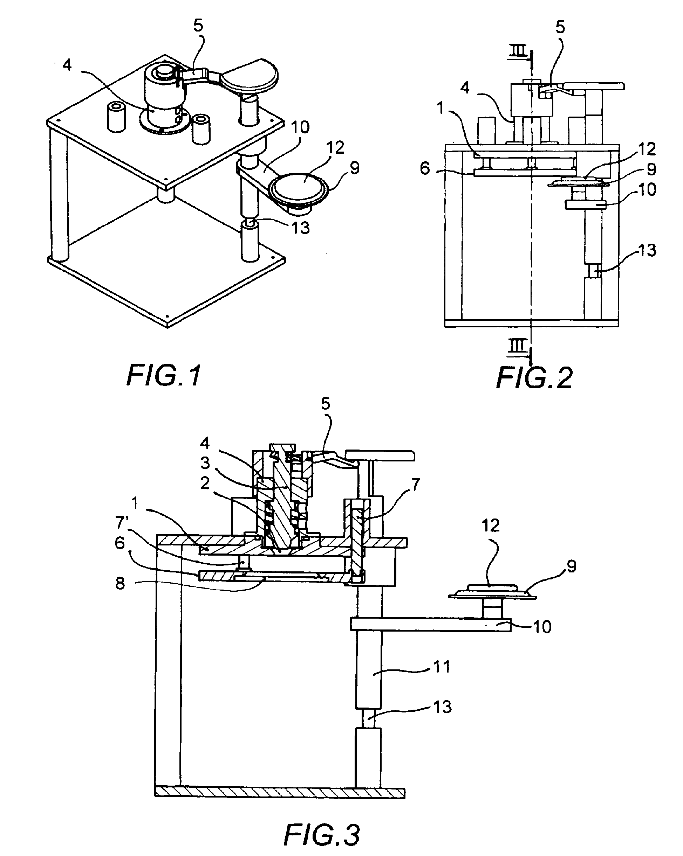

Pour des raisons de clarté, seule la figure 3, légèrement agrandie par rapport aux autres, comporte toutes les références.

L'appareil selon l'invention qui est représenté à titre d'exemple sur les dessins comporte essentiellement un plateau supérieur fixe 1 muni d'un orifice de soufflage 2 obturable, ici par une pointe d'obturation 3 montée dans un corps 4, tandis que des moyens d'alimentation en gaz, non représentés, sont prévus pour injecter du gaz tel que de l'air dans cet exemple, par les orifices du corps 4 visibles sur les dessins.

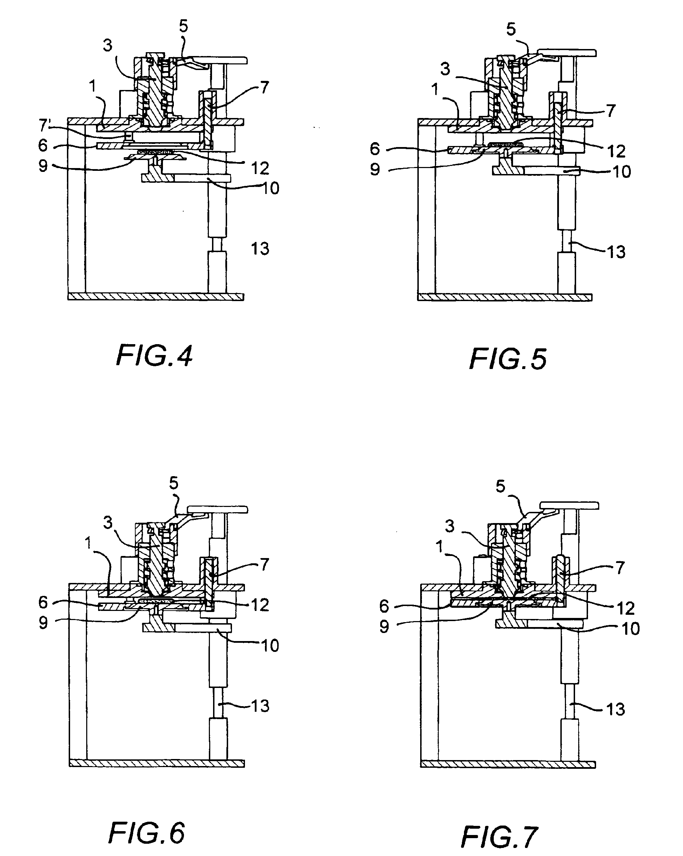

On comprend que la pointe 3 soumise à un moyen élastique peut être actionnée par un bras 5 pour la disposer, soit dans une position dans laquelle l'orifice 2 est libre (figures 3, 4, 8, 9 et 10), ou au contraire, dans une position d'obturation partielle (figure 5) ou d'obturation totale (figures 6 et 7).

L'appareil comporte une couronne 6 qui est montée mobile en translation de manière à pouvoir se rapprocher par un actionneur dont il sera question ci-après, et réciproquement s'écarter du plateau supérieur 1 au moyen par exemple ici d'au moins un actionneur 7, 7'.

Dans l'exemple représenté, on a même prévu trois actionneurs dont seuls les actionneurs 7 et 7' sont visibles. Chaque actionneur 7, 7' comporte, par exemple, une tige de guidage muni d'un ressort pour solliciter la couronne 6 à s'écarter et un moyen magnétique (par exemple une ventouse magnétique) pour le maintien en position, mais on peut bien sûr utiliser d'autres types d'actionneurs.

La couronne 6 détermine un ajour central 8 en forme de disque.

Une platine support 9 est disposée à l'extrémité d'un bras 10 monté rotatif sur un arbre 11 (par exemple au moyen d'une came, comme il sera précisé ci-après).

La platine 9 sensiblement à l'horizontal est destinée à recevoir un échantillon de pâte sous la forme d'un disque de pâte 12.

Un actionneur 13 (vérin électrique à came, électro-aimant, ...) est prévu dans l'arbre 11 afin de mouvoir en translation le bras 10 et par le fait la platine 9 et comme il sera précisé ci-après, la couronne 6 et même le bras 5.

Par ailleurs, ladite platine 9 est dimensionnée de manière complémentaire et conjuguée de l'ajour 8 de façon à le boucher afin de former avec la couronne 6 un plateau inférieur (6, 9) complet, comme le montrent les figures 5, 6 et 7, et dont il sera question ci-après. On peut également constater que dans cet exemple et comme représenté, la platine 9 présente un épaulement correspondant à la forme conjuguée de l'ajour 8 de la couronne 6, assurant ainsi un guidage et un calage de la platine 9 dans ledit ajour 8.

En position initiale représentée sur les figures 1, 2 et 3, le disque de pâte 12 est disposé sur la platine support 9 de manière manuelle ou robotisée, le bras 10 étant pivoté vers l'extérieur pour faciliter l'opération.

Les phases suivantes d'analyse sont parfaitement illustrées sur les figures suivantes.

Le bras 10 pivote de manière à positionner la platine support 9 sous la couronne 6, comme le montre la figure 4, au moyen, par exemple, d'une came dont est muni l'actionneur 13.

L'actionneur 13 permet en même temps de rapprocher le bras 10 et donc la platine support 9 vers la couronne 6 jusqu'à ce que la platine support 9 vienne boucher l'ajour 8 et compléter ainsi la couronne 6 pour former un plateau inférieur (6, 9), comme le montre bien la figure 5.

Comme le montrent les figures 6 et 7, le plateau inférieur (6, 9) ainsi formé et le bras 10 sont entraînés en translation par l'actionneur 13 de manière à rapprocher ledit plateau inférieur (6, 9) vers le plateau supérieur 1 jusqu'à ce que le disque de pâte 12 soit écrasé entre lesdits plateaux (1 ; 6, 9), comme le montre bien la figure 7, les actionneurs 7, 7' assurant le guidage en translation dudit plateau inférieur.

On remarquera par ailleurs qu'au cours du mouvement du plateau inférieur (6, 9) illustré sur les figures 5 à 7, le bras d'actionnement 5, commandé ici par l'actionneur 13, aura progressivement poussé la pointe 3 jusqu'à ce que celle-ci obture complètement l'orifice de soufflage 2, comme le montrent les figures 6 et 7, de telle sorte que le disque de pâte 12 puisse être écrasé entre deux plateaux complets ne présentant aucun orifice.

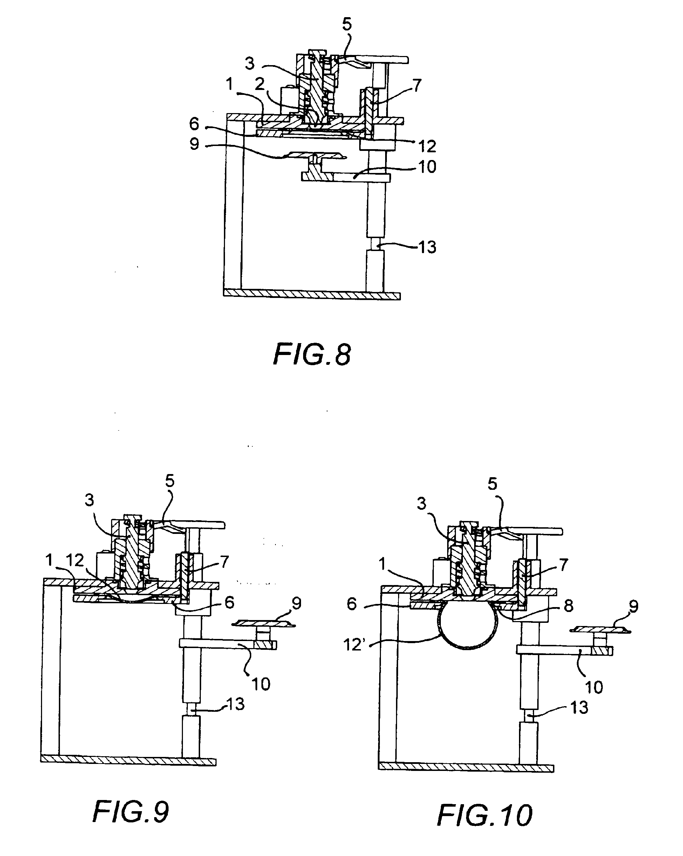

La figure 8 montre que la platine 9 est ensuite écartée de la couronne 6 au moyen de l'actionneur 13, ce qui libère son ajour central 8 et l'espace situé sous une partie du disque de pâte 12, tandis que la couronne 6 est maintenue dans cette position par les actionneurs 7, 7', de telle sorte que le disque de pâte 12 reste ainsi pincé à sa périphérie entre le plateau 1 et la couronne 6. Sur cette figure 8, on peut également constater que la pointe 3 libère l'orifice 2.

Les figures 9 et 10 illustrent le pivotement du bras 10 vers l'extérieur et le soufflage du disque de pâte 12 sur sa face supérieure par l'orifice 2 jusqu'à former une bulle 12' (figure 10) qui s'étend ainsi vers le bas au travers de l'ajour 8.

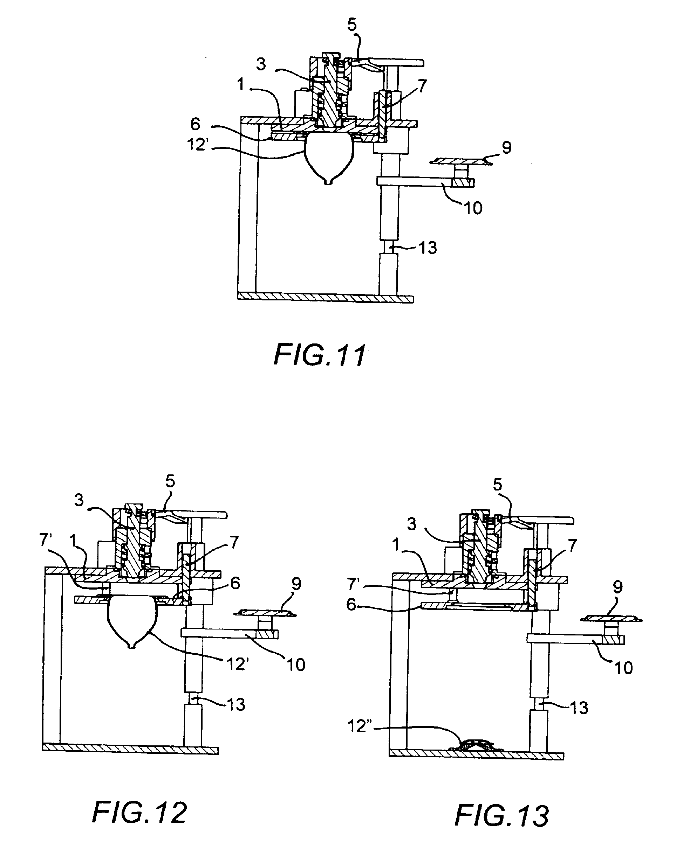

Cette bulle 12' est gonflée jusqu'à son éclatement schématisé sur les figures 11 et 12.

Après éclatement de la bulle 12', la couronne 6 s'écarte du plateau supérieur 1 au moyen des l'actionneur 7, 7', comme le montre bien la figure 12, au moyen, par exemple, des ressorts dont ils sont munis comme précisé ci-avant.

Le reste de l'échantillon de pâte après éclatement 12" peut ensuite être aisément retiré manuellement ou automatiquement, comme le schématise la figure 13.

Cette figure 13 montre en outre le retour du dispositif dans sa position initiale représentée sur les figures 1 à 3.

Par ailleurs, il est clair que l'on effectue une mesure de la pression de l'air dans la bulle 12' depuis son amorce schématisée sur la figure 9 jusqu'à son éclatement schématisé sur la figure 11.

La description qui précède d'un extensimètre, représenté dans plusieurs positions successives, illustre à la fois un mode de réalisation d'un dispositif et un procédé d'analyse selon l'invention donnés à titre d'exemple.

## Revendications

1. Procédé d'analyse par soufflage des caractéristiques d'un échantillon de pâte sous la forme d'un disque (12) qui est positionné sensiblement à l'horizontal, qui est préalablement écrasé entre deux plateaux, respectivement inférieur et supérieur, et pincé sur son pourtour et qui est soumis à une pression de gaz sur sa face supérieure pour former une bulle (12') de telle sorte que ladite bulle (12') se forme, par extension, vers le bas et qui est gonflée jusqu'à éclatement pendant que l'on enregistre la pression exercée, **caractérisé en ce qu'**on effectue le soufflage par un orifice (2) du plateau supérieur (1) et que l'on amène le disque de pâte (12) sous ledit plateau supérieur (1) au moyen d'une platine support (9) mobile que l'on positionne au centre d'une couronne (6) complémentaire de ladite platine (9) pour former avec celle-ci le plateau inférieur.

2. Procédé selon la revendication 1, **caractérisé en ce que** la platine support (9) est mue selon deux mouvements combinés de rotation et de translation en vue de permettre son positionnement et son mouvement d'écrasement du disque de pâte (12), ainsi que son dégagement pour libérer l'espace sous une partie du disque de pâte (12) afin de permettre son gonflage.

3. Extensimètre pour l'analyse par soufflage des caractéristiques mécaniques d'un échantillon de pâte sous la forme d'un disque (12), muni de moyens pour pincer le disque de pâte sur son pourtour, d'un moyen de mise sous pression d'un gaz pour former par soufflage une bulle (12') et de moyens d'enregistrement de la pression exercée, extensimètre **caractérisé en ce qu'**il comporte un plateau supérieur (1) fixe muni d'un orifice de soufflage (2) obturable et une couronne (6) qui détermine un ajour central (8) et qui est disposée sous le plateau supérieur (1), ladite couronne étant montée de manière translative par rapport audit plateau supérieur afin de pouvoir s'en rapprocher, être maintenue dans cette position, et respectivement s'en écarter, tandis qu'une platine support (9) mobile est prévue pour transporter le disque de pâte (12) sous ladite couronne (6), ladite platine support mobile étant dimensionnée pour venir dans l'ajour central (8) de la couronne et compléter celle-ci afin de former avec elle un plateau inférieur destiné à permettre l'écrasement du disque de pâte en se rapprochant du plateau supérieur (1).

4. Extensimètre selon la revendication 3, **caractérisé en ce que** la platine support (9) est munie d'un bras (10) actionné selon des mouvements de rotation et de translation de manière à permettre successivement, le positionnement de ladite platine (9) sous la couronne (6) et ensuite dans l'ajour central (8) de celle-ci, puis sa translation avec ladite couronne (6) vers le plateau supérieur (1) pour assurer l'écrasement du disque de pâte (12), au moins un moyen (7,7') étant prévu pour maintenir dans cette position la couronne (6), puis le dégagement de la platine support de ladite couronne afin de libérer ledit ajour central (8) et l'espace situé sous une partie du disque de pâte (12) qui peut ainsi rester pincé sur son pourtour entre le plateau supérieur (1) et la couronne (6) et subir un soufflage sur sa face supérieure par l'orifice (2) dudit plateau supérieur (1).

5. Extensimètre selon la revendication 4, **caractérisé en ce que** le bras (10) est entraîné en translation et en rotation par un actionneur (13) à came et **en ce que** le moyen (7,7') qui permet d'assurer le maintien en position rapprochée de la couronne (6) par rapport au plateau supérieur (1), est prévu pour permettre aussi d'assurer l'éloignement de ladite couronne (6) dudit plateau supérieur (1).

## Claims

1. A method for the analysis by blowing of the characteristics of a paste sample having the shape of a disk (12) positioned substantially horizontally, which is previously crushed between two respectively lower and upper plates, and pinched on its perimeter, and the upper face of which is exposed to a gas pressure in order to form a bubble (12'), so that said bubble (12') takes shape downwards by extension, and which is inflated up to the blowing out thereof, while the exerted pressure is recorded, **characterized in that** said blowing is carried out through a hole (2) provided in the upper plate (1) and **in that** the paste disk (12) is brought under said upper plate (1) using a mobile support plate (9) which is positioned at the centre of a complementary ring (6) of said support plate (9) in order to form the lower plate therewith.

2. A method according to claim 1, **characterized in that** the support plate (9) is moved along two combined rotational and translational motions with a view to enabling the positioning thereof and the motion thereof for crushing the paste disk (12), as well as the disengagement thereof in order to clear the space under a portion of the paste disk (12) so as to enable the inflation thereof.

3. An extensiometer for the analysis by blowing of the mechanical characteristics of a paste sample having the shape of a disk (12), provided with means for pinching the paste disk on the perimeter thereof, means for pressurizing a gas in order to form a bubble (12') by blowing, and means for recording the exerted pressure, the extensiometer being **characterised in that** it includes a stationary upper plate (1) provided with a sealable blowing hole (2) and a ring (6) which defines a central cut-out (8) and which is positioned under the upper plate (1), with said ring being translationally mounted with respect to said upper plate in order to be able to get closer thereto, to be held in this position and respectively to be moved away therefrom, whereas a mobile support plate (9) is provided for transporting the paste disk (12) under said ring (6), with said mobile support plate being so dimensioned as to come into the central cut-out (8) of the ring and complement it in order to form a lower plate therewith for enabling the crushing of the paste disk when getting closer to the upper plate (1).

4. An extensiometer according to claim 3, **characterized in that** the support plate (9) is provided with an arm (10) actuated along rotational and translational motions so as to successively enable the positioning of said support plate (9) under the ring (6) and then in the central cut-out (8) thereof, then the translation thereof with said ring (6) to the upper plate (1) in order to ensure the crushing of the paste disk (12), with at least one means (7, 7') being provided for holding the ring (6) in this position, then the disengagement of the support plate (9) from said ring in order to release said central cut-out (8) and the space located under a portion of the paste disk (12) which can thus be pinched on the perimeter thereof between the upper plate (1) and the ring (6) and the upper face of which can be exposed to blowing through the hole (2) of said upper plate (1).

5. An extensiometer according to claim 4, **characterized in that** the arm (10) is driven in translation and in rotation by a cam-operated actuator (13) and **in that** the means (7, 7') which holds the ring (6) in a position close to the ring (6) with respect to the upper plate (1) is also provided for moving said ring (6) away from said upper plate (1).

## Patentansprüche

1. Analyseverfahren per Gebläse der Merkmale einer Probe einer Masse in Form einer Scheibe (12), die deutlich horizontal positioniert ist, die zuvor zwischen zwei Platten, jeweils einer unteren und einer oberen, eingequetscht ist, und auf ihrem Umfang eingeklemmt ist und die auf ihrer oberen Seite einem Gasdruck unterzogen wird, um eine Blase (12) derart zu formen, dass die genannte Blase (12') sich durch Ausdehnung nach unten formt und die bis zum Platzen aufgebläht wird, während der ausgeübte Druck erfasst wird, **dadurch gekennzeichnet, dass** das Blasen durch eine Öffnung (2) der oberen Platte (1) durchgeführt wird und dass die Massenscheibe (12) unter der genannten oberen Platte (1) mittels einer mobilen Trägerplatine (9), die im Zentrum einer komplementären Krone (6) der genannten Platine (9) positioniert wird, um mit dieser die untere Platte zu formen, verbracht wird.

2. Verfahren gemäß Anspruch 1, **dadurch gekennzeichnet, dass** die Trägerplatine (9) gemäß zwei kombinierten Rotations- und Translationsbewegungen bewegt wird, um ihre Positionierung und ihre Quetschbewegung der Massenscheibe (12) sowie ihre Freisetzung zur Freigabe des Raums unter einem Teil der Massenscheibe (12) zu erlauben, um ihr Aufblähen zu erlauben.

3. Ausdehnungsmesser zur Analyse per Gebläse der mechanischen Merkmale einer Probe einer Masse in Form einer Scheibe (12), die mit Mitteln zum Einklemmen der Massenscheibe auf ihrem Umfang, einem Mittel zum UnterDrucksetzen eines Gases zur Bildung einer Blase (12') durch Blasen und Erfassungsmittel des ausgeübten Drucks ausgestattet ist, Ausdehnungsmesser, **dadurch gekennzeichnet, dass** er eine feste obere Platte (1) umfasst, die mit einer verschließbaren Gebläseöffnung (2) und einer Krone (6) versehen ist, die einen zentralen Durchbruch (8) bestimmt und die unter der oberen Platte (1) angeordnet ist, wobei die genannte Krone translativ im Verhältnis zur genannten oberen Platte angebracht ist, um sich ihr annähern zu können, in dieser Position gehalten werden zu können und sich jeweils davon entfernen zu können, während eine mobile Trägerplatine (9) vorgesehen ist, um die Massenscheibe (12) unter der genannten Krone (6) zu transportieren, wobei die genannte mobile Trägerplatine ausgelegt ist, um in einem zentralen Durchbruch (8) der Krone einzugreifen und diese zu vervollständigen, um mit ihr eine untere Platte zu formen, die dazu bestimmt ist, das Einquetschen der Massenscheibe zu erlauben, indem sie sich der oberen Platte (1) nähert.

4. Ausdehnungsmesser gemäß Anspruch 3, **dadurch gekennzeichnet, dass** die Trägerplatine (9) mit einem Arm (10) ausgerüstet ist, der gemäß den Rotations- und Translationsbewegungen derart betätigt wird, dass sukzessive die Positionierung der genannten Platte (9) unter der Krone (6) und anschließend in dem zentralen Durchbruch (8) derselben, dann ihre Translation mit der genannten Krone (6) zur oberen Platte (1) erlaubt werden, um das Einquetschen der Scheibenplatte (12) zu gewährleisten, wobei wenigstens ein Mittel (7, 7") vorgesehen ist, um die Krone (6), dann die Freisetzung der Trägerplatine der genannten Krone in dieser Position zu halten, um den genannten zentralen Durchbruch (8) und den Raum, der sich unter einem Teil der Massenscheibe (12) befindet, der somit auf seinem Umfang zwischen der oberen Platte (1) und der Krone (6) eingeklemmt bleiben und einem Blasen auf seiner oberen Seite durch die Öffnung (2) der genannten oberen Platte (1) unterzogen werden kann, freizusetzen.

5. Ausdehnungsmesser gemäß Anspruch 4, **dadurch gekennzeichnet, dass** der Arm (10) in Translation und in Rotation durch ein Betätigungsglied (13) mit Nocke angetrieben wird und dass das Mittel (7, 7"), das die Gewährleistung des Festhaltens in angenäherter Position der Krone (6) im Verhältnis zur oberen Platte (1) erlaubt, vorgesehen ist, um ebenfalls die Gewährleistung der Entfernung der genannten Krone (6) der oberen Platte (1) zu erlauben.
